# EUROPÄISCHE PATENTSCHRIFT

(11) **EP 1 439 801 B1**
(45) Veröffentlichungstag und Bekanntmachung des Hinweises auf die Patenterteilung: **01.07.2009**
(21) Anmeldenummer: 01978711.8
(22) Anmeldetag: 19.10.2001
(51) Int. Cl.: A61F 2/36, A61F 2/46

(54) **FEMURPROTHESE**
FEMORAL PROSTHESIS
PROTHESE DE FEMUR

(43) Veröffentlichungstag der Anmeldung: 28.07.2004
(73) Patentinhaber: Biomet Merck GmbH, 3216 Ried b. Kerzers (CH)
(72) Erfinder: BURETTE, Jean, Luc, B-4221 Neuville-en-Condroz (BE); KOCH, Rudolf, CH-4436 Oberdorf (CH)
(74) Vertreter: Rosenich, Paul
(86) Internationale Anmeldenummer: PCT/IB2001/001966
(87) Internationale Veröffentlichungsnummer: WO 2003/034955

(56) Entgegenhaltungen:
- EP-A- 0 781 532
- FR-A- 2 705 558
- FR-A- 2 710 835
- FR-A- 2 766 355
- FR-A- 2 779 342
- US-A- 4 787 909

## Beschreibung

Die Erfindung betrifft eine Femurprothese für den Einsatz in einem Femur. Fernurprothesen müssen gegenüber dem Femur in allen Achsrichtungen eine bestimmte Befestigungsstabilität und in Drehrichtung um die Femurachse eine gewisse Drehstabilität aufweisen, um eine optimale Rekonstruktion der Hüftgelenksgeometrie zu ermöglichen.

Aus der FR-A-2 766 355 ist eine Oberschenkelprothese bekannt. Es wird darin ein Prothesen-Schaft mit einem konisch zulaufenden medullären Abschnitt, einem Halsteil und einem Kopf für die Aufnahme einer Gelenksverbindung geoffenbart. Dieser Prothesen-Schaft hat eine Bohrung quer durch seine metaphysische Zone in der Frontal- Ebene, wobei diese Bohrung etwa 45 Grad gegen die Längs-Achse des Schafts geneigt ist. Weiters hat dieser Schaft eine Reihe anderer Bohrungen in zwei aufeinander senkrecht stehenden Ebenen, die einen wesentlichen Teil des medullärer Abschnitts abdecken. Die Bohrungen im medullären Abschnitt sind in Abständen von 1,5 cm voneinander getrennt und liegen in Frontal- und Saggital-Ebenen. Die Bohrungen dienen zur Aufnahme von Knochenschrauben und zur Befestigung des Prothese-Schafts im Oberschenkelknochen.

Aus der EP-A-0781532 ist eine implantierbare Vorrichtung, im besonderen eine Hüflgelenkkopf- Prothese bekannt Diese Prothese hat einen aus biokompatiblern Material bestehenden Körper der einen inneren Hohlraum bildet und offene Durchbrechungen aufweist, die vom Holraum nach außen führen. Der Hohlraum dient zur Aufnahme von Spongiosa oder anderem biologischen Material, in das das Gewebe, das die implantierte Vorrichtung umgibt, durch die Durchbrechungen hindurch hineinwachsen soll. Die Vorrichtung ist mit mindestens zwei Elektroden versehen, von denen mindestens eine im Hohlraum mit Abstand von der den Hohlraum bildenden Innenseite des Körpers angeordnet ist. Die Elektroden sind mit einer Anordnung zum Zuführen einer niederfrequenten Wechselspannung versehen, sodass durch die zugeführte Spannung innerhalb des Hohlraumes ein niederfrequentes elektrisches Wechselfeld und ein niederfrequenter Wechselstrom entstehen, wodurch das Gewebewachstum gefördert wird.

Insbesondere bei Revisionsprothesen versucht man, die an und für sich grosse Oeffnung im Femur mit Füllmaterial zu verdichten, um durch eine relativ stark konisch ausgebildete Prothese eine gute Revisionsprothetik zu ermöglichen. Insbesondere bei Revisionsprothesen, aber auch fallweise bei herkömmlichen Prothesen, ist es vorgesehen oder zu berücksichtigen, dass sich die Prothese in Axialrichtung nach dem Einsetzen geringfügig nachsenkt.

Prothesen mit Kragen, die am stirnseitig angeschnittenen Trochanter abgestützt werden, können zwar nicht beliebig absinken, jedoch können sie im Trochanterbereich durch Überlastung Knochenabbau bewirken. Die Kragenabstützungen können ferner das angestrebte primär zur Verfestigung der Verankerung notwendige Nachsetzen beeinträchtigen und somit zu Schaftlockerungen führen.

Vor allem bei Revisionsmassnahmen ist ausserdem gerade im Trochanterbereich eine für Prothetik ungenügende Knochenaufbau-Situation gegeben.

Dies ist vor allem auch für die Verdrehsicherung von Prothesen ein Problem, da diese somit im Trochanterbereich unter Umständen nicht mehr optimal gegen Verdrehung verankert werden können.

Zur Erhöhung der Verdrehsicherung von Prothesen wurden bisher verschiedene Massnahmen vorgeschlagen: Im Schnitt vierkantig aufgebaute Prothesen verfügen über eine hohe Eigendrehsicherung. Prothesen mit radial abragenden Lamellen sind ebensogut drehgesichert, wobei jedoch die Lamellen zwangsläufig stark von radial her in den Knochen einschneiden. Das später bei Revisionen allenfalls notwendige Entfernen solcher tief eingeschnittener Prothesen ist häufig schwierig, weshalb diese Art von Verdrehsicherung nicht immer angewendet werden kann.

Der Erfindung liegt somit die Aufgabe zugrunde, eine neuartige Femurprothese insbesondere für Revisionszwecke zu schaffen, die einerseits einen guten Sitz in axialer Richtung, jedoch in der Option auf ein geringfügiges axiales Nachgeben einer eingebauten Prothese und mit einer ausgezeichneten Verdrehsicherung zu schaffen.

Gelöst wird die Aufgabe durch die Herstellung einer entlang ihrer Achse relativ stark konisch gestalteten Prothese, die über wenigstens ein radial erstrecktes durchgehendes Langloch verfügt, wobei das Langloch für die Aufnahme einer Querschraube ausgebildet ist, die beim Einbau der Prothese von aussen durch die Wand des Femur, durch das Langloch und durch die gegenüberliegende Wand des Femur gebohrt bzw. eingeschraubt wird. Das Langloch übernimmt somit zusammen mit der Schraube eine in axialer Richtung dynamisierte Verdrehsicherung.

Anstelle einer Schraube könnte es sich auch um einen Bolzen oder dergleichen handeln, der im Knochen auf eine andere als die angegebene Art befestigt ist und sich im montierten Zustand durch das Langloch erstreckt.

Die konische Ausbildung der Prothese erlaubt einerseits eine gute axiale Abstützung, insbesondere am eingebrachten Füllmaterial im Falle einer Revisionsprothetik, während Langloch und Schraube für eine optimale Verdrehsicherung sorgen. Dadurch, dass die Schraube in einem Langloch platziert ist, erlaubt dieses ein relatives Spiel zwischen der Prothese und der Schraube, was ein geringfügiges Nachsinken der Prothese bis zum Anschlag der Schraube am oberen Langlochende erlaubt. Durch diesen Anschlag kann somit auch ein übermässiges Absinken der Prothese begrenzt werden, was hinsichtlich der symmetrischen Ausbildung von Prothesen für das linke und rechte Bein eines Patienten vorteilhaft sein kann.

Zur Verbesserung der Drehsicherung kann der Schaft der Prothese noch mit radial abragenden Rippen versehen sein, die beispielsweise durch U-förmige Nuten voneinander getrennt sind und in axialer Richtung verlaufen.

Im Gegensatz zu anderen bekannten Femurprothesen weist die erfindungsgemässe Prothese jedenfalls keine stützende Schulter und keinen Flansch auf.

Gemäss einer besonderen Ausgestaltung verfügt die Prothese über zwei in axialer Richtung übereinander angeordnete Langlöcher, die insbesondere in der proximalen Hälfte des Schaftes angeordnet sind und in einem Winkel von ca. 60° bzw. ca. (40°-80°) zueinander stehen.

Dieser Winkel ermöglicht das Einsetzen von zwei Befestigungsschrauben an unterschiedlichen Femurstellen und erhöht somit die Verdrehsicherungswirkung und Dauerhaftigkeit derselben, ohne den Prothesenschaft übermässig zu schwächen.

Der wesentliche Vorteil der Verwendung von Langlöchern liegt in der Tatsache, dass eine Verdrehsicherung gewährleistet ist, aber noch eine Bewegungs- / Anpassungsmöglichkeit der Prothese in ihrer Längsrichtung gegeben ist. Auf diese Weise wird ein Sich-Setzen der Prothese, gefolgt von einem guten Einwachsen unterstützt.

Im Falle einer optimalen Ausbildung des Konus und der bevorzugt vorgesehenen zusätzlichen radialen Rippen dienen die Schrauben lediglich einer sofort wirksamen vorübergehenden Verdrehsicherung, die im Zuge des Einwachsens der Prothese im Femur anschliessend durch den Prothesenschaft selbst übernommen werden kann.

Einer besonderen Ausgestaltung der Erfindung zufolge wird zur Montage der Schrauben eine an sich bekannte, jedoch besonders ausgebildete Schrauben-Positioniereinrichtung vorgesehen. Diese Einrichtung dient einerseits als Bohrlehre für das gewebeschonende, minimalinvasive Führen eines Knochenbohrers und andererseits zum lagerichtigen Einschieben und Eindrehen der Befestigungsschrauben. Die Positioniereinrichtung berücksichtigt die vorhin erwähnten verschiedenen Platzierungsorte der Schrauben oder Bolzen durch vordefinierte Einstell- bzw. Einrastmöglichkeiten.

Die Offenbarungsgehalte der Zeichnungen, ebenso wie die weiteren in den Ansprüchen beschriebenen bzw. unter Schutz gestellten Details, gehören mit zum Offenbarungsgehalt der Anmeldung.

Weitere Ausbildungen der Erfindung und Varianten dazu sind in den weiteren abhängigen Patentansprüchen angegeben.

### Figurenbeschreibung

Die Figurenbeschreibung und die Bezugszeichenliste bilden eine Einheit, die sich durch die übrigen Teile der Beschreibung und Ansprüche im Sinne einer vollständigen Offenbarung gegenseitig ergänzen. Gleiche Bezugszeichen bedeuten gleiche Bauteile; Bezugszeichen mit gleichen Nummern, jedoch unterschiedlichen Indizes bedeuten unterschiedliche Bauteile mit gleichen Funktionen beziehungsweise gleichen oder ähnlichen Aufgaben.

Die Figuren werden übergreifend und zusammenhängend beschrieben und sind nur beispielhaft und nicht zwingend proportional richtig dargestellt.

Da sie lediglich Ausführungsbeispiele darstellen, schränken die Figuren und die Figurenbeschreibung die Erfindung nicht ein.

Hierbei zeigen schematisch dargestellt
- Fig. 1 -: eine erfindungsgemässe Femurprothese,
- Fig. 2 -: als Detail die Langlöcher der Prothese nach Fig. 1,
- Fig. 3 -: den Aufbau der Femurprothese im Trochanterbereich,
- Fig. 4 -: den Aufbau der Femurprothese im Trochanterbereich aus einer anderen Ansicht,
- Fig. 5 -: den Aufbau der Positioniereinrichtung an einem Femurknochen und
- Fig. 6 -: eine Prothese und eine zerlegte Positioniereinrichtung.

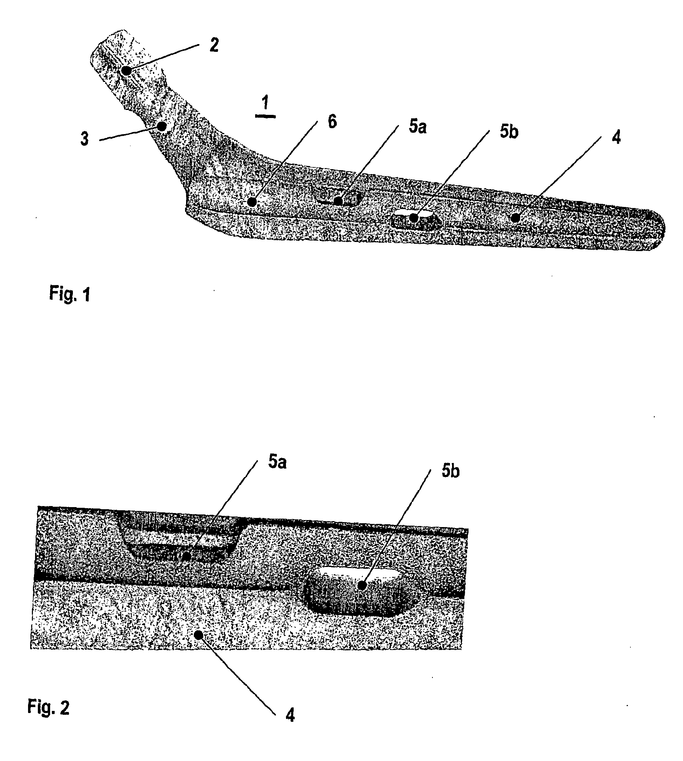

In Fig. 1 ist eine erfindungsgemässe Femurprothese 1 dargestellt. Hierbei erkennt man einen Konus 2 zur Aufnahme eines nicht dargestellten, an sich bekannten, sphärischen Kugelkopfes und einen speziell ausgeformten Hals 3. Ein Schaft 4 ist konisch ausgeformt und weist auf seiner gesamten Länge parallel angeordnete U-förmige Nuten 6 und Langlöcher 5a und 5b, die gegeneinander entlang der Längsachse der Prothese auf dem Schaft 4, aber auch um die Längsachse um ca. 60 Grad versetzt sind, auf.

In Fig. 2 ist in einer vergrösserten Detaildarstellung die Anordnung der Langlöcher 5a und 5b auf dem Schaft 4 gezeigt.

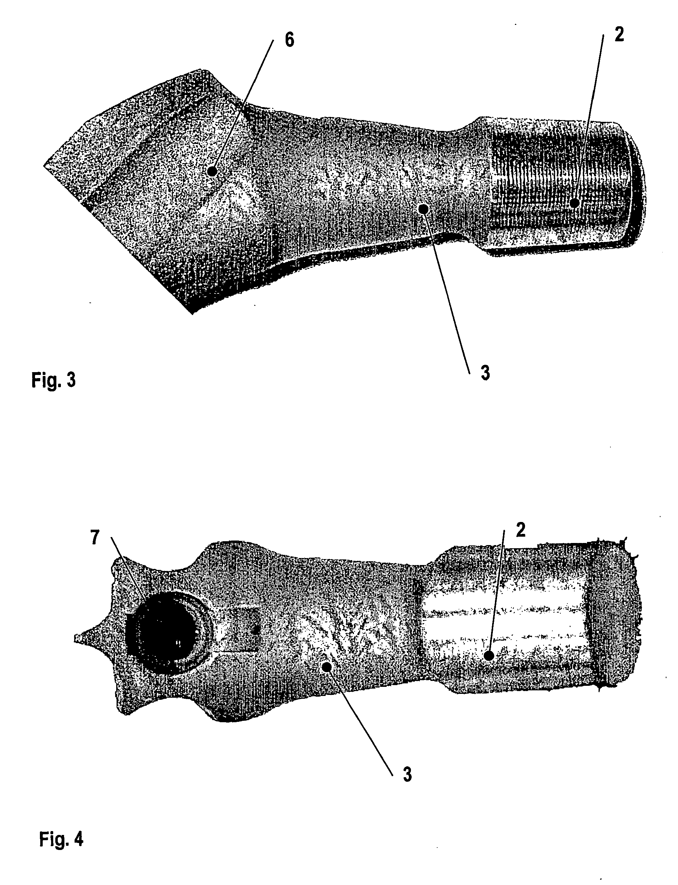

Fig. 3 zeigt den sogenannten Trochanterbereich mit dem Konus 2, dem Hals 3 und der U-förmigen Nut 6.

Fig. 4 ist eine Draufsicht auf den Trochanterbereich, die ein Einschlagloch 7 zeigt. Das Einschlagloch verfügt über ein Innengewinde, in dem eine erfindungsgemässe oder herkömmliche Positioniereinrichtung fixierbar ist.

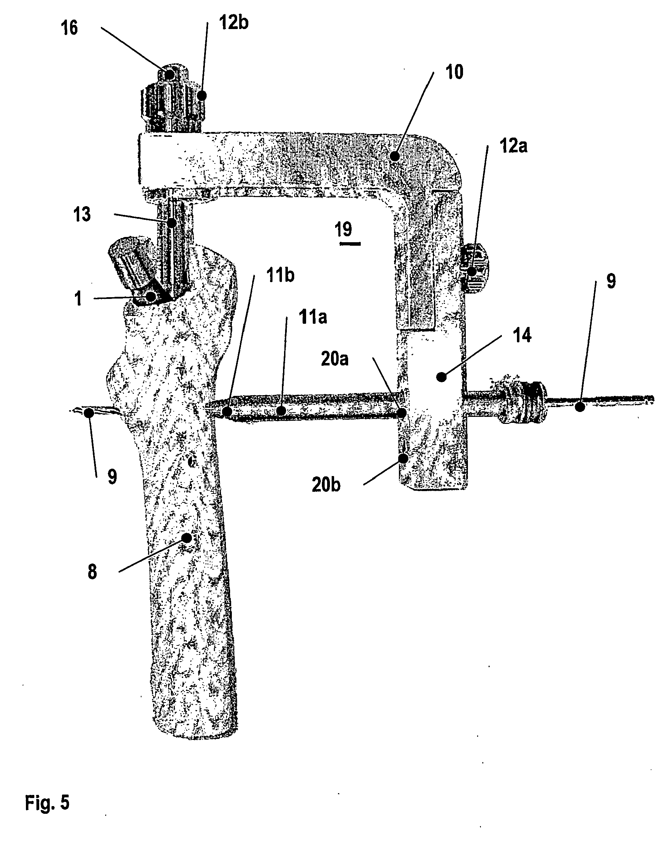

Fig. 5 zeigt die Positioniereinrichtung 19 im montierten Zustand, aufgesetzt mittels eines Gewindebolzens 16, welcher gegen einen Führungsbolzen 13 verschraubt und mit einer Fixiermutter 12b einen Zielbügel 10 hält.

Gemäss einer bevorzugten Ausgestaltung der Positioniereinrichtung 19 ist die Befestigung des Zielbügels 10 am Führungsbolzen 13 so schwenkbar gehalten, dass, ohne ein Positionierteil 14 auswechseln zu müssen, sich in einem oberen Führungsloch 20a eine Zwangsführung zum oberen Langloch 5a der Prothese 1 ergibt und im unteren Führungsloch 20b (nach Schwenken des Zielbügels in eine vordefinierte Position auf dem Führungsbolzen 13) eine Zwangsführung zum unteren Langloch 5b der Prothese 1 ergibt.

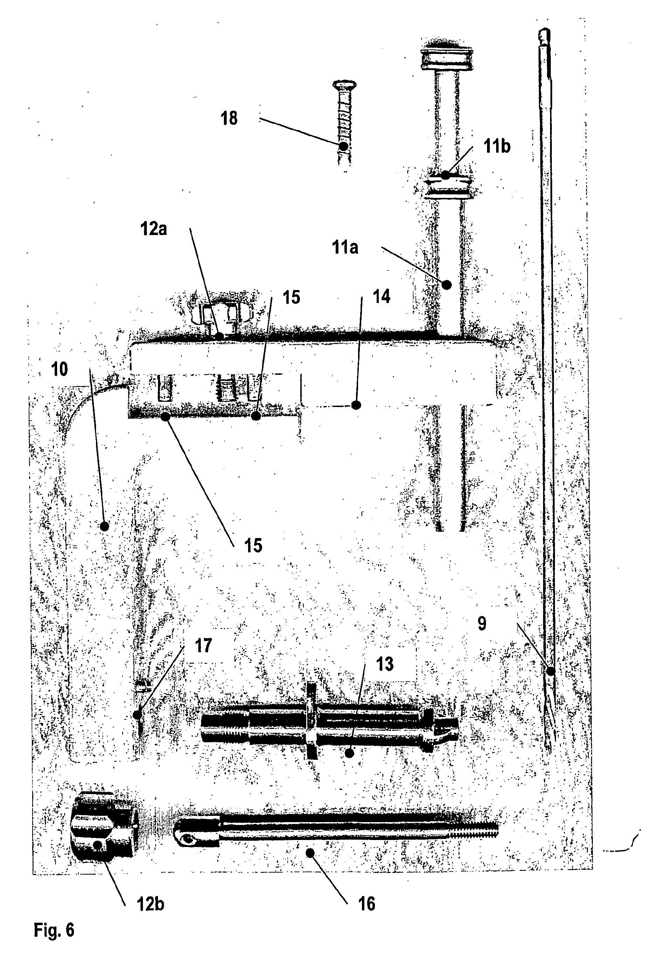

In Fig. 6 sind die Prothese und die zerlegte Positioniereinrichtung dargestellt. Hierbei ist zu erkennen, dass verschiedene vordefinierte Anfahrpositionen für einen Bohrer 9 entweder durch verschieden gestaltete auswechselbare Positionierteile 14 oder gemäss vorliegender bevorzugten Ausgestaltung mittels einem Positionierteil 14 erreichbar sind. Dieses Positionierteil 14 ist mit Haltestiften 15 und einer Sicherungsschraube 12a an dem Zielbügel 10 befestigt. Dadurch, dass zwischen Zielbügel 10 und dem Positionierteil 14 eine Trennstelle vorgesehen ist, können auch je nach Bedarf längere oder kürzere Prothesen mit den entsprechend längeren oder kürzeren auswechselbaren Positionierteilen 14 zum Einsatz gelangen. Ferner können die Schraubenpositionen wahlweise zwischen dem distalen Ende der Schaftbohrungen 5a und 5b sowie deren Zentrum axial verschoben werden, was zu einer einstellbaren Nachsink-Begrenzung des Schaftes 4 dient.

Der Zielbügel 10 ist über den Gewindebolzen 16, welcher in dem Einschlagloch 7 der Prothese 1 angelenkt ist, dem Führungsbolzen 13 und der Fixiermutter 12b drehbar gehalten und rastet mittels einem oder mehreren Positionsstiften 17 und entsprechenden Bohrungen in einer Schulter des Führungsbolzens 13 in den vordefinierten Stellungen ein.

Des Weiteren sind konzentrische Bohrhülsen 11a und 11b dargestellt, durch die der Bohrer 9, aber auch der Einsatz einer Knochenschraube 18 mit einem nicht gezeichneten Schraubenzieher geführt wird.

### Bezugszeichenliste

1-Prothese
2-Konus
3-Hals
4-Schaft
5a, b-Langloch
6-U-förmige Nut
7-Einschlagloch
8-Femurknochen
9-Bohrer
10-Zielbügel
11a, b-Bohrbüchse
12a-Fixierschraube
12b-Fixiermutter
13-Führungsbolzen
14-Positionierteil
15-Haltestift
16-Gewindebolzen
17-Positionsstift
18-Knochenschraube
19-Positioniereinrichtung
20a, b-Führungsloch

## Patentansprüche

1. Femurprothese **(1)** mit einem Konus **(2)** und einem Schaft **(4),** welcher wenigstens ein Langloch **(5)** aufweist, **dadurch gekennzeichnet, dass** die Femurprothese mindestens eine Querschraube **(18)** umfasst, mithilfe derer der Schaft **(4)** durch das Langloch **(5)** mit Hilft einer Positioniereinrichtung **(19)** mit dem Femur **(8)** verschraubbar ist.

2. Femurprothese nach Anspruch 1, **dadurch gekennzeichnet, dass** das Langloch (5) in der proximalen Hälfte des Schaftes **(4)** liegt

3. Femurprothese nach einem der vorhergehenden Ansprüche, **dadurch gekennzeichnet, dass** der Schaft **(4)** wenigstens zwei Langlöcher **(5)** aufweist, die zueinander in einem Winkel von ca. 40-80 Grad, insbesondere von ca. 60 Grad ausgerichtet sind.

4. Femurprothese nach einem der vorhergehenden Ansprüche, **dadurch gekennzeichnet, dass** der Schaft **(4)** konisch ausgeformt ist, wobei die Konizität ca. 2-8 Grad, vorzugsweise ca. 5 Grad beträgt.

5. Femurprothese nach einem der vorhergehenden Ansprüche, **dadurch gekennzeichnet dass** der Schaft **(4)** mit längs verlaufenden, parallel angeordneten U-förmigen Nuten **(6)** versehen ist.

6. Femurprothese nach einem der vorhergehenden Ansprüche, **dadurch gekennzeichnet, dass** die Oberfläche des Schaftes **(4)** knochengewebefreundlich nachbehandelt oder ausgebildet ist.

7. Femurprothese nach einem der vorhergehenden Ansprüche, **dadurch gekennzeichnet, dass** sie mit einem Einschlagloch **(7)** ausgestattet ist, wo gegebenenfalls unter anderem über einen Anschluss, z.B. ein Gewinde eine Positioniereinrichtung **(19)** befestigbar ist

8. Femurprothese nach einem der vorhergehenden Anspruche, **dadurch gekennzeichnet, dass** sie eine abnehmbare Positioniereinrichtung **(19)** umfasst, die so ausgestaltet ist, dass mindestens ein Langloch **(5)** mit einer vordefinierten Position der Positioniereinrichtung **(19)** korrespondiert.

9. Femurprothese nach Anspruch 8, **dadurch gekennzeichnet, dass** die Positioniereinrichtung **(19)** Einrastpositionen umfasst, die vordefinierten Stellungen durch Einrasten ermöglicht, welche durch Schwenken eines Zielbügels **(10)** erreichbar sind.

10. Femurprothese nach Anspruch 8, **dadurch gekennzeichnet, dass** ein Positionierteil **(14)** mit Bohrungen **(20a und 20b)** versehen ist, welche eine mit den Langlöchern **(5a und 5b)** korrespondierende Zwangsführung von Bohrhülsen **(11a und 11b)** ergeben.

11. Femurprothese nach Anspruch 8, **dadurch gekennzeichnet, dass** die Positioniereinrichtung **(19)** auswechselbare, unterschiedlichen Positionierteile **(14)** umfasst, die vordefinierte Stellungen durch ihren Einsatz erreichbar macht.

12. Femurprothese nach Anspruch 8, **dadurch gekennzeichnet dass** die Positioniereinrichtung **(19)** unterschiedlich lange, auswechselbare Positionierteile **(14)** versieht, sodass unterschiedlich lange Prothesen **(1)** einsetzbar sind.

## Claims

1. Femoral prosthesis (1) comprising a cone (2) and a shank (4) with at leas one slot (5), **characterized in that** the femoral prosthesis comprises at least one transverse screw (18) by means of which the shank (4) can be screwed to the femur (8) though the slot (5) with the aid of a positioning device (19).

2. Femoral prosthesis according to Claim 1, **characterized in that** the slot (5) is located in the proximal half of the shank (4).

3. Femoral prosthesis according to one of the preceding claims, **characterized in that** the shank (4) has at least two slots (5) which are oriented at an mangle of approximately 40-80 degrees, in particular approximately 60 degrees, to one another.

4. Femoral prosthesis according to one of the preceding claims, **characterized in that** the shank (4) has a conical design, the conicity bering approximately 2-8 degrees, preferably approximately 5 degrees.

5. Femoral prosthesis according to one of the preceding claims, **characterized in that** U-shaped grooves (6), expending in the longitudinal direction and arranged in parallel, are provided on the shank (4).

6. Femoral prosthesis according to one of the preceding claims, **characterized in that** the surface of the shank (4) is designed to be bone-tissue friendly or has undergone finishing treatment to that effect.

7. Femoral prosthesis according to one of the preceding claims, **characterized in that** it is equipped with a striking-in hole (7) where a positioning device (19) can be attached, possible, inter alia, by means of a connector, for example a thread.

8. Femoral prosthesis according to one of the preceding claims, **characterized in that** it comprises a removable positioning device (19) which is designed such that at least one slot (5) corresponds to a redefined position of the positioning device (19).

9. Femoral prosthesis according to Claim 8, **characterized in that** the positioning device (19) comprises latching positions which enable redefined position by latching, which positions are reachable by pivoting a target frame (10).

10. Femoral prosthesis according to Claim 8, **characterized in that** a positioning part (14) is provided with bores (20a and 20b) which lead to forced guiding of drill sleeves (11a and 11b), the forced guiding corresponding to the slots (5a and 5b).

11. Femoral prosthesis according to Claim 8, **characterized in that** the positioning device (19) comprises interchangeable, different positioning parts (14) whose use makes predefined positions reachable.

12. Femoral prosthesis according to Claim 8, **characterized in that** the positioning device (19) provides interchangeable positioning parts (14) of different lengths so that prostheses (1) with different lengths can be used.

## Revendications

1. Prothèse (1) du fémur qui présente un cône (2) et une tige (4) qui présente au moins un trou oblong (5),
**caractérisée en ce que**
la prothèse du fémur comporte au moins une vis transversale (18) à l'aide de laquelle la tige (4) peut être vissée sur le fémur (8) à travers le trou oblong (5) au moyen d'un dispositif de positionnement (19).

2. Prothèse du fémur selon la revendication 1, **caractérisée en ce que** le trou oblong (5) est situé dans la moitié proximale de la tige (9).

3. Prothèse du fémur selon l'une des revendications précédentes, **caractérisée en ce que** la tige (4) présente au moins deux trous oblongs (5) qui sont orientés l'un par rapport à l'autre sous un angle d'environ 40 à 80 degrés et en particulier d'environ 60 degrés.

4. Prothèse du fémur selon l'une des revendications précédentes, **caractérisée en ce que** la tige (4) a une forme clonique, sa conicité étant d'environ 2 à 8 degrés et de préférence d'environ 5 degrés.

5. Prothèse du fémur selon l'une des revendications précédentes, **caractérisée en ce que** la tige (4) est dotée de rainures (6) qui s'étendent longitudinalement, disposées parallèlement et en forme de U.

6. Prothèse du fémur selon l'une des revendication précédentes, **caractérisée en ce que** pour être compatible avec le tissu osseux, la surface de la tige (4) reçoit un traitement de finition ou est configurée dans ce but.

7. Prosthèse du fémur selon l'une des revendication précédentes, **caractérisée en ce qu'**elle est dotée d'un trou de battage (7) où un dispositif de positionnement (19) peut être fixé, éventuellement entre autres par l'intermédiaire d'un raccord, par exemple un filet.

8. Prothèse du fémur selon l'une des revendications précédente, **caractérisée en ce qu'**elle comprend un dispositif de positionnement (19) amovible configuré de telle sorte qu'au moins un trou oblong (5) corresponde à une positron prédéfinie du dispositif de positionnement (19).

9. Prothèse du fémur selon la revendication 8, **caractérisée en ce que** le dispositif de positionnement (19) comprend des positions d'encliquetage qui permettent par encliquetage d'atteindre des positons prédéfinies par pivotement d'un étrier de visée (10).

10. Prothèse du fémur selon la revendication 8, **caractérisée en ce qu'**une pièce de positionnement (14) est dotée d'alésages (20a et 20b) qui définissent un guidage forcé de douilles d'alésage (11a et 11b) en correspondance aux trous oblongs (5a et 5b).

11. Prothèse du fémur selon la revendication 8, **caractérisée en ce que** le dispositif de positionnement (19) comprend des pièces de positionnement (14) remplaçables et différentes dont l'utilisation permet d'obtenir des positions prédéfinies.

12. Prothèse du fémur selon la revendication 8, **caractérisée en ce que** le dispositif de positionnement (19) prévoit des pièces de positionnement (14) remplaçables de différentes longueurs, ce qui permet d'utiliser des prothèses (1) de différentes longueurs.
